# EUROPEAN PATENT SPECIFICATION

(11) **EP 4 598 723 B1**
(45) Date of publication and mention of the grant of the patent: **11.11.2026**
(21) Application number: 23793045.8
(22) Date of filing: 02.10.2023
(51) Int. Cl.: B29B 17/02, B07C 5/342, D01G 11/00, B29L 31/00

(54) **METHOD FOR RECOVERING TEXTILE FIBRES FROM DISCARDED TEXTILE ITEMS AND ASSOCIATED PLANT**
VERFAHREN ZUR RÜCKGEWINNUNG VON TEXTILFASERN AUS ENTSORGTEN TEXTILARTIKELN UND ZUGEHÖRIGE ANLAGE
PROCÉDÉ DE RÉCUPÉRATION DE FIBRES TEXTILES À PARTIR D'ARTICLES TEXTILES MIS AU REBUT ET INSTALLATION ASSOCIÉE

(30) Priority: 04.10.2022 IT 202200020394
(43) Date of publication of application: 13.08.2025
(73) Proprietor: IGERS S.R.L., 20123 Milano (IT)
(72) Inventor: MICELI, Gian Luca, 20154 Milano (IT)
(74) Representative: Bellomia, Paolo
(86) International application number: PCT/IB2023/059853
(87) International publication number: WO 2024/074974

(56) References cited:
- WO-A2-2006/036850
- CN-A- 105 665 299
- CN-B- 106 245 125
- JP-A- 2009 191 375
- DAMAYANTI DAMAYANTI ET AL: "Possibility Routes for Textile Recycling Technology", POLYMERS, vol. 13, no. 21, 1 November 2021 (2021-11-01), CH, pages 3834, XP055946051, ISSN: 2073-4360, DOI: 10.3390/polym13213834

## Description

### Technical Field

The present invention relates to a method and plant for recovering textile fibres from discarded textile items.

In particular, the present invention relates to a method and a plant for recovering textile fibres from discarded textile items for the textile, non-woven (TNT), clothing, PPE, automotive and construction industries.

### Prior art

The terminology "discarded textile items" herein refers to textile items to be discarded of various origins, including, for example:
- urban collection through skips for post-consumer items (used, endof-life, non-reusable garments),
- unsold items (unsold garments and left-over stock), sorted/waste items already sorted for the vintage and second-hand market, and
- industrial textile residues (mattress cuttings, mould cuttings, negatives, selvedges).

It is well known to fray industrial textile residues to obtain TNT - and sometimes staple material - but recovery is not carried out nowadays from urban collection and unsold/discarded items, as it is complex to implement as well as economically unsustainable.

In fact, the sorting by material type and the manual removal of non-woven material (i.e. the rigid parts of natural, synthetic, ferrous material, such as buttons, zips, sliders, zippers) makes the process time-consuming, complicated and expensive.

In order to verify the composition of the garment and carry out a rigorous sorting process, an operator is required to read the attached label, where present and comprehensible (people often remove them because they are annoying, or they become discoloured over time and become illegible), so it is clear that the recovery process requires long lead times and high personnel costs in order to be efficient in terms of production volume. Furthermore, even if the operator were experienced and specialised, he could still not process garments whose origin and composition cannot be certified.

In addition, the items may not be frayed without first removing the rigid parts, otherwise the textile material could catch fire during fraying due to sparks produced by friction between the fraying heads and the rigid metal parts. The removal of the rigid parts is then carried out by hand during the manual sorting of the garment.

To date, therefore, not all the discarded textile items can be properly processed to be recovered, due to the heterogeneous composition thereof, the presence of rigid elements, too expensive sorting costs, and due to lack of technology; therefore, many discarded items end up in landfills or are converted into secondary fuels, emitting environmentally harmful microplastic particles into the atmosphere.

However, some regulations provide that in the coming years textile waste will no longer be allowed to be sent to waste-to-energy plants, landfill sites or plants to produce secondary solid fuel, so anything that cannot be recovered for the vintage or second-hand market (because it is threadbare and worn out) and production waste, will have to be processed/recovered in another way.

Document CN106245125B discloses an example of a known plant for the recovery of textile fibres from discarded textile items. Documents CN105665299A and JP2009191375A disclose example of known sorting methods and apparatus. Document WO2006/036850A2 discloses an example of a clothes tumbler with ozone generator.

### Object of the invention

In this context, the technical task underlying the present invention is therefore to propose a method and a plant for recovering textile fibres from discarder textile items that overcome one or more of the drawbacks of the prior art mentioned above.

In particular, the object of the present invention is to make available a method for recovering textile fibres from discarded textile items that makes it possible to effectively and efficiently recover different types of discarded textile items in a cost-effective and sustainable manner.

A further object of the present invention is to propose a method for recovering textile fibres from discarded textile items that allows to make the item sorting and processing automated in a fast, accurate and efficient manner.

The object of the present invention is also to propose a plant for recovering textile fibres from discarded textile items that allows to manage the entire recovery process automatically, improving the efficiency of the production process, and to quickly and efficiently create recovered staple material to be used as yarn in industrial processes.

The specified technical task and the specified objects are substantially achieved by a method and a plant for recovering textile fibres from discarded textile items, comprising the technical characteristics set forth in one or more of the appended claims. The present invention is defined by the method for recovering textile fibres from discarded textile items as comprised in claim 1, and by the plant as comprised in claim 6.

In particular, in a first aspect, the present invention provides a method for recovering textile fibres from discarded textile items comprising the steps of:
- storing discarded textile items, such as garments or industrial textile residues, at a storage station,
- moving the items to an analysis station,
- analysing the items by NIR *(Near-InfraRed)* spectroscopy to detect characteristic parameters of the item textile fibres,
- comparing the characteristic parameters detected from each item analysed with reference characteristic parameters,
- moving a selection of items, whose textile fibres have characteristic parameters that fulfil predetermined acceptability criteria, towards a processing station,
- processing said selection of items in the processing station, so as to separate any rigid or semi-rigid parts of the plastic and/or metal type, such as buttons and zips, from the textile material of the selection of items, and fray the textile material to obtain recovered textile fibres.

Advantageously, the items are then analysed in an automated manner to precisely and accurately identify the type of existing textile fibres and are consequently sorted according to a pre-established rationale to be processed. In particular, mechanised and automated processing involves a sub-step of removing the rigid or semi-rigid parts from the items and a subsequent step of fraying the remaining textile material so that this last operation can be carried out efficiently and safely.

Thanks to the selection and sorting rationale based on the characteristic parameters of the textile fibres analysed, it is possible to efficiently and automatically recover all the discarded textile items stored according to pre-established operating rationales based on the recovery campaigns, in order to create new recovered textile fibres that can be used for new industrial applications.

Advantageously, the method allows to recover discarded textile items, used garments and production/warehouse waste, avoiding their disposal in landfills and waste-to-energy plants.

In a second aspect, the invention provides a plant for recovering textile fibres from discarded textile items, comprising:
- a storage station suitable for receiving discarded textile items, such as garments or industrial textile residues,
- an analysis station comprising at least one NIR spectrometer configured to detect characteristic parameters of the textile fibres of the items,
- a processing station comprising a separating unit configured to separate any rigid or semi-rigid parts of the plastic and/or metal type, such as buttons and zips, from the textile material of the items, and a fraying unit configured to mechanically fray the separated textile material to obtain recovered textile fibres,
- handling means configured to move said items from the storage station to the processing station via the analysis station, and
- a management and control unit configured to compare the characteristic parameters detected from each item analysed with reference characteristic parameters, and to command the movement of a selection of items, whose textile fibres have characteristic parameters that fulfil predetermined acceptability criteria, towards the processing station.

Advantageously, the presence of the analysis station allows items to be analysed quickly and accurately, and the management and control unit enables the analysed items to be efficiently sorted in order to produce preferably homogenous selections of items to be processed to obtain preferably homogenous recovered textile fibres according to pre-established rationales and specific recovery requirements. Advantageously, the plant therefore allows to process any textile waste from any source in a single solution with no need for manual sorting before or after storage, automatically managing the items that can be processed simultaneously.

The processing station allows selected items to be mechanically processed in cascade to first remove the rigid or semi-rigid parts for a given recovery campaign and only then to fray them to obtain the recovered fibres ready for further processing, with no need for manual intervention by operators but by means of appropriate separation and fraying devices that allow process time and costs to be reduced.

The dependent claims herein incorporated for reference, correspond to different embodiments of the invention.

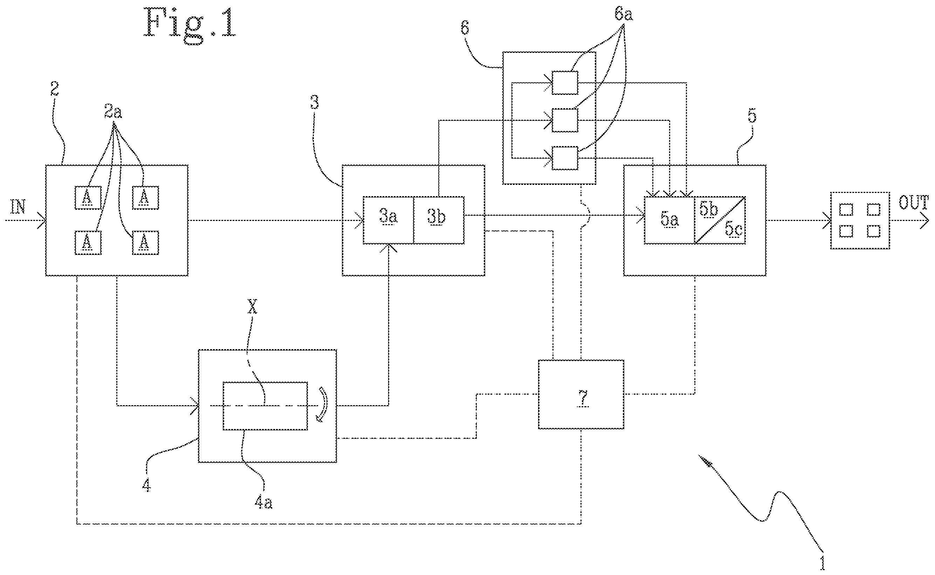

Further features and advantages of the present invention will become clearer from the indicative, and therefore non-limiting, description of a preferred, but not exclusive, embodiment of a method and plant for recovering textile fibres from discarded textile items, as shown in the diagram in Figure 1.

### Brief description of the drawings

Figure 1 is a diagram of the operation of a method and plant for recovering textile fibres from discarded textile items according to the present invention.

### Detailed description of preferred embodiments of the invention

With reference to the enclosed figures, a plant for recovering textile fibres from discarded textile items A has been globally referred to as 1.

The discarded textile items A may comprise garments such as: used, flawed, worn-out, non-reusable garments, stocks, or industrial textile residues such as: offcuts, scraps, negatives, selvedges, etc.

The items A comprise a portion of textile material and may comprise a portion of rigid or semi-rigid plastic and/or metal parts, e.g. buttons, zips, zippers, sliders, etc.

The method subject of in the present invention relates to the recovery of textile fibres from the textile portion of items A, preferably by using the plant 1, which will be described hereinafter.

The method comprises first of all a step of storing items A in a storage station 2. The items A stored in the storage station 2 may have different origins, but the method advantageously does not require manual sorting of incoming IN items A, which can therefore also be stored indiscriminately. Preferably, however, the method may provide a step of manually presorting the items A to separate any items A for *second-hand* use.

The method comprises a step of moving the items A to an analysis station 3.

The method comprises a step of sanitising the items A before they are moved towards the analysis station 3, wherein the items A are moved within an ozone sanitisation station 4. Ozone sanitising is advantageously carried out to remove bad smells, such as smoke, sweat, humidity or to eradicate moths, mites, viruses and bacteria present in the items A.

The sanitising step comprises also a sub-step of rotating an ozone sanitising cabin 4a within which the items A to be sanitised are arranged.

Thanks to the rotation of the cabin 4a it is possible to increase the ozone exposure surface area of each item A in order to effectively break down impurities on the items A, as the items A are continuously shaken/tilted due to the rotation of the cabin 4a.

The method comprises a step of analysing the items (coming from the storage station 2 or sanitising station 4) by NIR spectroscopy to detect characteristic parameters of the textile fibres of the items A.

NIR spectroscopy is essentially based on the physical phenomenon of the absorption of electromagnetic radiation by the analysed matrix. The resulting analytical signal is created by the interaction between the analysed sample and the radiation incident thereon.

The use of such technology makes it possible to analyse the items in less time, replacing conventional analytical/manual sorting techniques, increasing the precision, accuracy and speed of the process and reducing costs significantly.

The method provides a step of comparing the characteristic parameters measured from each analysed item A with reference characteristic parameters.

Preferably the detectable characteristic parameters are the chemical compositions of the materials and/or the material colour codes, preferably both of them.

The method thus comprises a step of moving a selection of items A, whose textile fibres have characteristic parameters that fulfil predetermined acceptability criteria (corresponding, for example, to the identification of "white cotton" items, where e.g. cotton must be present in an amount of no less than 95% and the shade of white must fall within a certain colour code range), to a processing station 5.

The method then comprises a step of processing the selection of items A with two sub-steps:
- separating any rigid or semi-rigid plastic and/or metal parts from the textile material of the selection of items A, and
- fraying the separated textile material to obtain recovered textile fibres.

Advantageously, thanks to the present invention, it is not only possible to efficiently recover any item A through separation and cascade fraying, but it is also possible to select homogeneous items A and process them simultaneously to obtain high quality homogeneous textile fibres based on the required recovery campaigns.

In fact, the method preferably comprises a step of accumulating within an accumulation station 6 selections of different items A, whose textile fibres have characteristic parameters that fulfil respective predetermined acceptability criteria according to a pre-established rationale.

It is thereby possible to first process one selection of items A and then another, depending on production requirements.

Preferably, moreover, the accumulation is carried out up until reaching a certain amount of accumulated items A (e.g. a certain weight or volume) of at least one selection of items A intended for the production of recovered textile fibres with homogeneous characteristics.

After the accumulation step, a subsequent step of moving at least a selection of accumulated items A to the processing station 5 is provided, preferably upon reaching the specified amount of accumulated items. Advantageously, it is thereby possible to guarantee a continuous recovery method, so that the processing of items A is never interrupted regardless of their origin, by processing one or more accumulated selections if, for example, a first selection is not in a sufficient amount to guarantee continuous processing without stopping the production process.

The presence of an accumulation station 6 makes it possible to have a lung/buffer of analysed and sorted items A if there are not enough processing lines capable of accommodating all types of analysed items A at the same time.

It is therefore possible, for example, to accumulate white cotton up until the capacity of the storage container 6a is reached, and in the meantime process the rest of the analysed items A that are not white cotton. When the limit volume of white cotton items accumulated in the container 6a is reached, they can be sent to the processing station 5 to be processed simultaneously.

Preferably the method further comprises a step of cleaning the processing station 5 to remove residues of previously processed items A before moving the accumulated items A towards said processing station 5. Advantageously, the cleaning step avoids possible contaminations of material other than that to be processed, with a view to guaranteeing a final product (recovered textile fibres) that can fulfil the required production requirements.

Preferably, the method may further comprise a step of mixing the recovered textile fibres, even more preferably textile fibres recovered by the method according to the present invention, after fraying with further recovered textile fibres, to obtain a mix of recovered textile fibres corresponding to certain acceptability requirements.

According to a further aspect of the present invention, a plant 1 for recovering textile fibres from discarded textile items A is provided, comprising:
- a storage station 2 suitable for receiving items A of discarded textile material, such as garments or industrial textile residues,
- an analysis station 3 comprising at least one NIR spectrometer 3a configured to detect characteristic parameters of the textile fibres of the items A,
- a processing station 5 comprising a separating unit 5a configured to separate any rigid or semi-rigid parts of the plastic and/or metal type, such as buttons and zips (hereinafter simply "rigid parts"), from the textile material of the items A and a fraying unit 5b configured to fray the separated textile material to obtain recovered textile fibres,
- handling means configured to move the items A from the storage station 2 to the processing station 5 via the analysis station 3, and
- a management and control unit 7 configured to compare the characteristic parameters detected from each analysed item A with reference characteristic parameters, and to command the movement of a selection of items A, whose textile fibres have characteristic parameters that fulfil predetermined acceptability criteria, towards the processing station 5.

Preferably, the storage station 2 comprises a plurality of storage containers 2a.

Preferably, the NIR spectrometer 3a is arranged inside a flight cabin, not shown, within which the items A to be analysed are fed and is provided with at least two detection lenses (or scanners, sensors) arranged in cascade, each one capable of detecting a certain characteristic parameter (e.g. chemical composition of the textile material and colour).

According to a possible embodiment of the plant, the analysis station 3 preferably comprises a plurality of NIR spectrometers 3a configured to analyse different characteristic parameters simultaneously. Advantageously, the management and control unit 7 is provided with a database of characteristic reference parameters with which a wide range of materials and colours are registered.

Preferably, the analysis station 3 comprises a sorting unit 3b configured to sort the analysed items A according to a pre-established rationale based on the predetermined acceptability criteria.

The sorting unit 3b may preferably comprise a compressed-air ejector configured to eject with a jet of compressed air an analysed item A that fulfils certain acceptability criteria and send it directly to the processing station 5, or to the accumulation station 6 depending on the operation rationale of the plant, as will become clear later.

Preferably, the separating unit 5a comprises a rotating drum provided with spikes distributed on the inner surface that are configured to interfere with the rigid parts of the items A during rotation/centrifugation of the items A within the drum, thus mechanically separating the rigid parts from the textile material.

Preferably, upstream of the separating unit 5a, the processing station 5 may comprise a shredder, not shown, configured to perform a first shredding step to reduce the volume of the items A.

Preferably, the handling means also comprise a pneumatic vacuum cleaner, not shown, configured to suck the textile material from the separating unit 5a and transfer it to the fraying unit 5b.

Preferably, the fraying unit 5b comprises a plurality of rotating drums with staves and spikes, not shown, configured to interfere with the textile material of the items A, received from the separating unit 5a, during rotation/centrifugation to open the textile material thereby mechanically fraying the textile fibres contained therein.

Depending on the type of items A and textile material to be processed, and on the quality of the textile fibres to be obtained, n cycles can be performed on the drums of the fraying unit 5b.

The plant 1 comprises a sanitising station 4, interposed between the storage station 2 and the analysis station 3, wherein a sanitising cabin 4a is arranged for sanitising the items A comprising a containment drum suitable for receiving the items A to be sanitised, said sanitising being carried out by means of ozone.

The handling means are configured to move the items A from the storage station 2 to the sanitising station 4 and from the sanitising station 4 to the processing station 3.

The containment drum is rotatable about a rotation axis X coincident with its main development axis.

Unlike the known sanitising cabins, which are static, the present sanitising cabin 4a is therefore of the dynamic type, making it possible to better sanitise the items A that are moved during the rotation of the containment drum.

Preferably, the plant comprises an accumulation station 6, interposed between the analysis station 3 and the processing station 5, wherein a plurality of accumulation containers 6a are arranged that are configured to receive different selections of analysed items A, whose textile fibres have characteristic parameters that fulfil respective predetermined acceptability criteria, according to a pre-established rationale.

Advantageously, the management and control unit 7 is configured to selectively control the movement of the respective selections of items A to the processing station 3, e.g. upon reaching a certain amount of items A in a storage container 6a, as explained above.

Preferably, moreover, the management and control unit 7 is configured to control and manage the operation of the sorting device 3b to automatically move the selected items A according to the pre-established rationale to the respective accumulation containers 6a.

Preferably, the system comprises a cleaning device, not shown in the attached figures, configured to remove item residues from the separating unit 5a and the fraying unit 5b.

Preferably, the processing station 5 comprises a mixer 5c, arranged downstream of the fraying unit 5b, configured to mix the recovered textile fibres after fraying with additional recovered textile fibres to obtain a mix of recovered textile fibres corresponding to predetermined acceptability requirements.

The mixer 5c allows fibres to be mixed together to obtain qualitative and replicable yarns that comply with records and parameters dictated by specific production requirements.

The present invention achieves the proposed objects, overcoming the drawbacks complained of in the prior art and providing an innovative process and plant in the field of textile waste recovery to recover the fraction of discarded textile material contained in items A typically intended for disposal, shredding or mixing with other plastics to create secondary solid fuel RDF for cement and waste-to-energy plants, and instead obtain staple fibre for use as yarn in industrial processes or TNT for less valuable applications.

## Claims

1. A method for recovering textile fibres from discarded textile items, comprising the steps of:
- storing discarded textile items (A), such as garments or industrial textile residues, at a storage station (2),
- moving said items (A) towards an analysis station (3),
- analysing said items (A) by means of NIR spectroscopy to detect characteristic parameters of the textile fibres of the items (A),
- comparing the characteristic parameters detected from each item (A) analysed with reference characteristic parameters,
- moving a selection of items (A), whose textile fibres have characteristic parameters that fulfil predetermined acceptability criteria, towards a processing station (5),
- processing said selection of items (A) in said processing station (5), so as to separate any rigid or semi-rigid parts of the plastic and/or metal type, such as buttons and zips, from the textile material of said selection of items (A), and tear said textile material to obtain recovered textile fibres;
wherein said method comprises a step of sanitising said items (A) before being moved to the analysis station (3), said sanitising step being carried out by moving said items (A) within an ozone sanitising station (4),
wherein said sanitising step comprises a sub-step of rotating an ozone sanitising cabin (4a) within which the items (A) to be sanitised are arranged.

2. The method according to claim 1, wherein said characteristic parameters are: chemical compositions of the materials and/or identification codes of the colours of the materials.

3. The method according to one of the preceding claims, comprising a step of accumulating within an accumulation station (6) different selections of items (A), whose textile fibres have characteristic parameters that fulfil respective predetermined acceptability criteria according to a pre-established rationale, preferably until an accumulated given amount of items (A) of at least one selection of items (A) is reached; and a subsequent step of moving said at least one accumulated selection of items (A) towards said processing station (5), preferably upon reaching said given amount of items (A).

4. The method according to claim 3, comprising a step of cleaning the processing station (5) to remove residues of previously processed items (A) before moving the accumulated items (A) towards said processing station (5).

5. The method according to one or more of the preceding claims, comprising a step of mixing the recovered textile fibres after fraying with additional recovered textile fibres to obtain a mixture of recovered textile fibres corresponding to predetermined acceptability requirements.

6. A plant (1) for the recovery of textile fibres from discarded textile items, comprising:
- a storage station (2) suitable for receiving items (A) of discarded textile material, such as garments or industrial textile residues,
- an analysis station (3) comprising at least one NIR spectrometer (3a) configured to detect characteristic parameters of the textile fibres of the items (A),
- a processing station (5) located downstream of the analysis station (3) and configured to process the items (A) received by the analysis station (3), said processing station (5) comprising a separating unit (5a) configured to separate any rigid or semi-rigid parts of the plastic and/or metal type, such as buttons and zips, from the textile material of the items (A), and a fraying unit (5b) configured to fray the separated textile material to obtain recovered textile fibres,
- handling means configured to move said items (A) from the storage station (2) to the processing station (5) via the analysis station (3), and
- a management and control unit (7) configured to compare the characteristic parameters detected from each item (A) analysed with reference characteristic parameters, and to command the movement of a selection of items (A), whose textile fibres have characteristic parameters that fulfil predetermined acceptability criteria, towards the processing station (5);
wherein said plant (1) comprises a sanitising station (4), interposed between the storage station (2) and the analysis station (3), wherein a sanitising cabin (4a) is arranged for sanitising the items (A) comprising a containment drum suitable for receiving the items (A) to be sanitised, said sanitisation being carried out by means of ozone,
wherein said containment drum is rotatable about a rotation axis (X) coincident with its main development axis.

7. The plant (1) according to claim 6, wherein said characteristic parameters are: chemical compositions of the materials and/or identification codes of the colours of the materials.

8. The plant (1) according to one of claims 6-7, comprising an accumulation station (6), interposed between said analysis station (3) and said processing station (5), wherein a plurality of accumulation containers (6a) are arranged that are configured to receive different selections of items (A) analysed, whose textile fibres have characteristic parameters that fulfil respective predetermined acceptability criteria, according to a pre-established rationale; said management and control unit (7) being configured to selectively command the movement of the respective selections of items (A) towards the processing station (5).

9. The plant (1) according to one or more of claims 6-8, comprising a device (5) for cleaning the processing station configured to remove item residues from the separating unit (5a) and the fraying unit (5b).

10. The plant (1) according to one or more of claims 6-9, wherein said processing station (5) comprises a mixer (5c), arranged downstream of the fraying unit (5b), configured to mix the recovered textile fibres after fraying with additional recovered textile fibres to obtain a mix of recovered textile fibres corresponding to predetermined acceptability requirements.

11. The plant (1) according to any one or more of claims 6-10, wherein said analysis station (3) comprises a sorting unit (3b) configured to sort the analysed items (A) according to a pre-established rationale based on the predetermined acceptability criteria; said sorting unit (3b) preferably comprising a compressed-air ejector.

## Patentansprüche

1. Verfahren zur Rückgewinnung von Textilfasern aus ausgesonderten Textilartikeln, umfassend die folgenden Schritte:
- Lagern von ausgesonderten Textilartikeln (A) wie Kleidungsstücken oder industriellen Textilresten an einer Lagerstation (2);
- Bewegen der Artikel (A) in Richtung einer Analysestation (3);
- Analysieren der Artikel (A) mittels NIR-Spektroskopie, um charakteristische Parameter der Textilfasern der Artikel (A) zu erkennen;
- Vergleichen der bei jedem analysierten Artikel (A) erkannten charakteristischen Parameter mit charakteristischen Referenzparametern;
- Bewegen einer Auswahl von Artikeln (A), deren Textilfasern charakteristische Parameter aufweisen, die vorbestimmte Akzeptanzkriterien erfüllen, in Richtung einer Verarbeitungsstation (5);
- Verarbeiten der Auswahl von Artikeln (A) in der Verarbeitungsstation (5), sodass etwaige starre oder halbstarre Teile vom Kunststoff- und/oder Metalltyp wie Knöpfe und Reißverschlüsse von dem Textilmaterial der Auswahl von Artikeln (A) getrennt werden und das Textilmaterial aufgerissen wird, um rückgewonnene Textilfasern zu erhalten,
wobei das Verfahren einen Schritt des Hygienisierens der Artikel (A) vor deren Bewegung zu der Analysestation (3) umfasst, wobei der Hygienisierungsschritt durch Bewegen der Artikel (A) innerhalb eine Ozon-Hygienisierungsstation (4) durchgeführt wird,
wobei der Hygienisierungsschritt einen Teilschritt des Drehens einer Ozon-Hygienisierungskabine (4a), innerhalb derer die zu hygienisierenden Artikel (A) angeordnet sind, umfasst.

2. Verfahren nach Anspruch 1, wobei die charakteristischen Parameter folgende sind: chemische Zusammensetzungen der Materialien und/oder Identifikationscodes der Farben der Materialien.

3. Verfahren nach einem der vorhergehenden Ansprüche, umfassend einen Schritt des Akkumulierens verschiedener Auswahlen von Artikeln (A), deren Textilfasern charakteristische Parameter aufweisen, die jeweilige vorbestimmte Akzeptanzkriterien gemäß einer im Vorfeld festgelegten Logik erfüllen, innerhalb einer Akkumulationsstation (6), vorzugsweise bis eine vorgegebene akkumulierte Menge von Artikeln (A) mindestens einer Auswahl von Artikeln (A) erreicht ist, und einen anschließenden Schritt des Bewegens der mindestens einen akkumulierten Auswahl von Artikeln (A) in Richtung der Verarbeitungsstation (5), vorzugsweise nach Erreichen der vorgegebenen Menge von Artikeln (A).

4. Verfahren nach Anspruch 3, umfassend einen Schritt des Reinigens der Verarbeitungsstation (5), um Reste zuvor verarbeiteter Artikel (A) zu entfernen, bevor die akkumulierten Artikel (A) in Richtung der Verarbeitungsstation (5) bewegt werden.

5. Verfahren nach einem oder mehreren der vorhergehenden Ansprüche, umfassend einen Schritt des Mischens der rückgewonnenen Textilfasern nach dem Zerfasern mit zusätzlichen rückgewonnenen Textilfasern, um eine Mischung von rückgewonnenen Textilfasern zu erhalten, die vorbestimmten Akzeptanzanforderungen entspricht.

6. Anlage (1) zur Rückgewinnung von Textilfasern aus ausgesonderten Textilartikeln, umfassend:
- eine Lagerstation (2), die zum Aufnehmen von Artikeln (A) aus ausgesondertem Textilmaterial wie Kleidungsstücken oder industriellen Textilresten geeignet ist;
- eine Analysestation (3), umfassend mindestens ein NIR-Spektrometer (3a), das ausgelegt ist, um charakteristische Parameter der Textilfasern der Artikel (A) zu erkennen;
- eine Verarbeitungsstation (5), umfassend eine Trenneinheit (5a), die ausgelegt ist, um etwaige starre oder halbstarre Teile vom Kunststoff- und/oder Metalltyp wie Knöpfe und Reißverschlüsse von dem Textilmaterial der Artikel (A) zu trennen, und eine Zerfaserungseinheit (5b), die ausgelegt ist, um das getrennte Textilmaterial zu zerfasern, um rückgewonnene Textilfasern zu erhalten;
- Handhabungsmittel, die ausgelegt sind, um die Artikel (A) von der Lagerstation (2) zu der Verarbeitungsstation (5) über die Analysestation (3) zu bewegen, und
- eine Verwaltungs- und Steuereinheit (7), die ausgelegt ist, um die bei jedem analysierten Artikel (A) erkannten charakteristischen Parameter mit charakteristischen Referenzparametern zu vergleichen und die Bewegung einer Auswahl von Artikeln (A), deren Textilfasern charakteristische Parameter aufweisen, die vorbestimmte Akzeptanzkriterien erfüllen, in Richtung der Verarbeitungsstation (5) anzuweisen,
wobei die Anlage (1) eine Hygienisierungsstation (4) umfasst, die zwischen der Lagerstation (2) und der Analysestation (3) eingesetzt ist, wobei eine Hygienisierungskabine (4a) zum Hygienisieren der Artikel (A) angeordnet ist, umfassend eine Behältertrommel, die zum Aufnehmen der zu hygienisierenden Artikel (A) geeignet ist, wobei die Hygienisierung mittels Ozon durchgeführt wird,
wobei die Behältertrommel um eine Drehachse (X) drehbar ist, die mit ihrer Hauptentwicklungsachse zusammenfällt.

7. Anlage (1) nach Anspruch 6, wobei die charakteristischen Parameter folgende sind: chemische Zusammensetzungen der Materialien und/oder Identifikationscodes der Farben der Materialien.

8. Anlage (1) nach einem der Ansprüche 6-7, umfassend eine Akkumulationsstation (6), die zwischen der Analysestation (3) und der Verarbeitungsstation (5) eingesetzt ist, wobei eine Vielzahl von Akkumulationsbehältnissen (6a) angeordnet ist, die ausgelegt sind, um verschiedene Auswahlen von analysierten Artikeln (A) aufzunehmen, deren Textilfasern charakteristische Parameter aufweisen, die jeweilige vorbestimmte Akzeptanzkriterien gemäß einer im Vorfeld festgelegten Logik erfüllen, wobei die Verwaltungsund Steuereinheit (7) ausgelegt ist, um die Bewegung der jeweiligen Auswahlen von Artikeln in Richtung der Verarbeitungsstation (5) wahlweise anzuweisen.

9. Anlage (1) nach einem oder mehreren der Ansprüche 6-8, umfassend eine Vorrichtung (5) zum Reinigen der Verarbeitungsstation, die ausgelegt ist, um Artikelreste aus der Trenneinheit (5a) und der Zerfaserungseinheit (5b) zu entfernen.

10. Anlage (1) nach einem oder mehreren der Ansprüche 6-9, wobei die Verarbeitungsstation (5) einen Mischer (5c) umfasst, der stromabwärts der Zerfaserungseinheit (5b) angeordnet und ausgelegt ist, um die rückgewonnenen Textilfasern nach dem Zerfasern mit zusätzlichen rückgewonnenen Textilfasern zu mischen, um eine Mischung von rückgewonnenen Textilfasern zu erhalten, die vorbestimmten Akzeptanzanforderungen entspricht.

11. Anlage (1) nach einem oder mehreren der Ansprüche 6-10, wobei die Analysestation (3) eine Sortiereinheit (3b) umfasst, die ausgelegt ist, um die analysierten Artikel (A) nach einer im Vorfeld festgelegten Logik basierend auf den vorbestimmten Akzeptanzkriterien zu sortieren, wobei die Sortiereinheit (3b) vorzugsweise einen Druckluft-Ejektor umfasst.

## Revendications

1. Procédé de récupération de fibres textiles à partir d'articles textiles mis au rebut, comprenant les étapes suivantes :
- le stockage d'articles textiles mis au rebut (A), tels que des vêtements ou des résidus textiles industriels, dans une station de stockage (2),
- l'acheminement desdits articles (A) vers une station d'analyse (3),
- l'analyse desdits articles (A) au moyen de la spectroscopie NIR afin de détecter les paramètres caractéristiques des fibres textiles des articles (A),
- la comparaison des paramètres caractéristiques détectés pour chaque article (A) analysé à des paramètres caractéristiques de référence,
- l'acheminement d'une sélection d'articles (A), dont les fibres textiles présentent des paramètres caractéristiques répondant à des critères d'acceptabilité prédéterminés, vers une station de traitement (5),
- le traitement de ladite sélection d'articles (A) dans ladite station de traitement (5), de manière à séparer toutes les parties rigides ou semi-rigides de type plastique et/ou métal, telles que les boutons et les fermetures éclair, du matériau textile de ladite sélection d'articles (A), et à déchirer ledit matériau textile pour obtenir des fibres textiles récupérées ;
dans lequel ledit procédé comprend une étape de désinfection desdits articles (A) avant leur transfert vers la station d'analyse (3), ladite étape de désinfection étant réalisée en faisant passer lesdits articles (A) dans une station de désinfection à l'ozone (4),
dans lequel ladite étape de désinfection comprend une sous-étape consistant à faire tourner une cabine de désinfection à l'ozone (4a) à l'intérieur de laquelle sont disposés les articles (A) à désinfecter.

2. Procédé selon la revendication 1, dans lequel lesdits paramètres caractéristiques sont : les compositions chimiques des matériaux et/ou les codes d'identification des couleurs des matériaux.

3. Procédé selon l'une des revendications précédentes, comprenant une étape consistant à accumuler, au sein d'une station d'accumulation (6), différentes sélections d'articles (A) dont les fibres textiles présentent des paramètres caractéristiques qui satisfont à des critères d'acceptabilité prédéterminés respectifs selon une logique préétablie, de préférence jusqu'à ce qu'une quantité donnée accumulée d'articles (A) d'au moins une sélection d'articles (A) soit atteinte ; et une étape suivante consistant à acheminer ladite au moins une sélection accumulée d'articles (A) vers ladite station de traitement (5), de préférence lorsque ladite quantité donnée d'articles (A) est atteinte.

4. Procédé selon la revendication 3, comprenant une étape consistant à nettoyer la station de traitement (5) afin d'éliminer les résidus d'articles (A) précédemment traités avant de transférer les articles (A) accumulés vers ladite station de traitement (5).

5. Procédé selon l'une ou plusieurs des revendications précédentes, comprenant une étape consistant à mélanger les fibres textiles récupérées après effilochage avec des fibres textiles récupérées supplémentaires afin d'obtenir un mélange de fibres textiles récupérées répondant à des exigences d'acceptabilité prédéterminées.

6. Installation (1) destinée à la récupération de fibres textiles à partir d'articles textiles mis au rebut, comprenant :
- une station de stockage (2) adaptée pour recevoir des articles (A) de matière textile mise au rebut, tels que des vêtements ou des résidus textiles industriels,
- une station d'analyse (3) comprenant au moins un spectromètre NIR (3a) configuré pour détecter les paramètres caractéristiques des fibres textiles des articles (A),
- une station de traitement (5) comprenant une unité de séparation (5a) configurée pour séparer les éventuelles parties rigides ou semi-rigides de type plastique et/ou métal, telles que des boutons et des fermetures éclair, du matériau textile des articles (A), et une unité d'effilochage (5b) configurée pour effilocher le matériau textile séparé afin d'obtenir des fibres textiles récupérées,
- des moyens de manutention configurés pour déplacer lesdits articles (A) de la station de stockage (2) vers la station de traitement (5) en passant par la station d'analyse (3), et
- une unité de gestion et de commande (7) configurée pour comparer les paramètres caractéristiques détectés sur chaque article (A) analysé à des paramètres caractéristiques de référence, et pour commander le déplacement d'une sélection d'articles (A), dont les fibres textiles présentent des paramètres caractéristiques répondant à des critères d'acceptabilité prédéterminés, vers la station de traitement (5) ;
dans laquelle ladite installation (1) comprend une station de désinfection (4), intercalée entre la station de stockage (2) et la station d'analyse (3), dans laquelle une cabine de désinfection (4a) est disposée pour désinfecter les articles (A) et comprend un tambour de confinement adapté pour recevoir les articles (A) à désinfecter, ladite désinfection étant effectuée au moyen d'ozone, dans laquelle ledit tambour de confinement peut tourner autour d'un axe de rotation (X) coïncidant avec son axe de développement principal.

7. Installation (1) selon la revendication 6, dans laquelle lesdits paramètres caractéristiques sont : les compositions chimiques des matériaux et/ou les codes d'identification des couleurs des matériaux.

8. Installation (1) selon l'une des revendications 6 à 7, comprenant une station d'accumulation (6), intercalée entre ladite station d'analyse (3) et ladite station de traitement (5), dans laquelle sont disposés une pluralité de conteneurs d'accumulation (6a) configurés pour recevoir différentes sélections d'articles (A) analysés, dont les fibres textiles présentent des paramètres caractéristiques qui satisfont à des critères d'acceptabilité prédéterminés respectifs, selon une logique préétablie ; ladite unité de gestion et de commande (7) étant configurée pour commander de manière sélective le déplacement des sélections respectives d'articles (A) vers la station de traitement (5).

9. Installation (1) selon l'une ou plusieurs des revendications 6 à 8, comprenant un dispositif (5) de nettoyage de la station de traitement, configuré pour éliminer les résidus d'articles provenant de l'unité de séparation (5a) et de l'unité d'effilochage (5b).

10. Installation (1) selon l'une ou plusieurs des revendications 6 à 9, dans laquelle ladite station de traitement (5) comprend un mélangeur (5c), disposé en aval de l'unité d'effilochage (5b), configuré pour mélanger les fibres textiles récupérées après effilochage avec des fibres textiles récupérées supplémentaires afin d'obtenir un mélange de fibres textiles récupérées répondant à des exigences d'acceptabilité prédéterminées.

11. Installation (1) selon l'une ou plusieurs des revendications 6 à 10, dans laquelle ladite station d'analyse (3) comprend une unité de tri (3b) configurée pour trier les articles analysés (A) selon une logique préétablie fondée sur les critères d'acceptabilité prédéterminés ; ladite unité de tri (3b) comprenant de préférence un éjecteur à air comprimé.
